# EUROPEAN PATENT SPECIFICATION

(11) **EP 0 795 008 B1**
(45) Date of publication and mention of the grant of the patent: **27.08.2003**
(21) Application number: 95934723.8
(22) Date of filing: 25.10.1995
(51) Int. Cl.: C12N 5/22, A61L 27/00

(54) **HUMAN HYPERTROPHIC CHONDROCYTE CELL-LINES**
HUMANE HYPERTROPHE CHRONDROZYTEN ZELLLINIEN
LIGNEES CELLULAIRES DE CHONDROCYTES HYPERTROPHIQUES D' ORIGINE HUMAINE

(30) Priority: 13.12.1994 GB 9425071
(43) Date of publication of application: 17.09.1997
(60) Divisional application: 01110823.0
(73) Proprietor: CellFactors plc, Newport NP10 8UH (GB)
(72) Inventor: Stringer, Bradley Michael John, Cardiff CF2 6BS (GB)
(74) Representative: Davies, Jonathan Mark
(86) International application number: GB9502497
(87) International publication number: WO96018728

(56) References cited:
- WO-A-94/09118
- J CELL BIOL, JAN 1995, 128 (1-2) P239-45, UNITED STATES, LEFEBVRE V ET AL 'Type X collagen gene expression in mouse chondrocytes immortalized by a temperature-sensitive simian virus 40 large tumor antigen.'
- JOURNAL OF BIOLOGICAL CHEMISTRY, vol. 268, no. 13, May 1993 MD US, pages 9645-9652, MASAHIRO IWAMOTO ET AL. 'Expression and role of c-myc in chondrocytes undergoing endochondral ossification'
- PROC. NATL. ACAD. SCI. U. S. A., 1993, 3289-93, MALLEIN-GERIN, FREDERIC ET AL 'Expression of simian virus 40 large T (tumor) oncogene in mouse chondrocytes induces cell proliferation without loss of the differentiated phenotype'
- INT. J. CANCER, vol. 48, 1991 pages 717-725, TAKIGAWA, MASAHARU ET AL. 'Establishement from a human chondrosarcoma of a new immortal cell line with high tumorigenicty in vivo, which is able to form proteoglycan-rich cartilage-like nodules and to respond to insulin in vitro'
- DEVELOPMENTAL BIOLOGY, vol. 149, 1992 pages 168-176, QUARTO, RODOLFO ET AL. 'Constitutive myc expression impairs hypertrophy and calcification in cartilage'

## Description

The invention relates to Human Hypertrophic Chondrocyte Cell-Lines.

In addition, the invention relates to hypertrophic chondrocytes which may be of particular advantage in the development of new biomaterials for use in replacing skeletal tissues such as hips and joints.

Bone formation is characterised by calcification in the region of hypertrophic chondrocytes. Whilst vascular invasion is important for materialisation in vivo, it is clear that initiation of calcification can be independent of vascularization, since hypertrophic chondrocytes can spontaneously mineralise their matrix *in vitro* in the absence of exogenous phosphate.

In J. Cell. Biol., January 1995, 128 (1-2) p239-245, Lefebvre., *et al,* discloses Type X collagen gene expression in mouse chondrocytes immortalized by a temperature sensitive simian virus 40 large tumour antigen. Several clones of immortalized cells were isolated after retroviral infection of rib primary chondrocytes prepared from 1 to 3 day old mice. At the time of their isolation (day 0) and after 5 days in primary culture, mouse chondrocytes were fully differentiated and the expressed phenotype indicated that a significant proportion of the cells were hypertrophic.

We have produced chondrocyte cell-lines from foetal tissue which express type X collagen, a marker of hypertrophic chondrocytes.

Such chondrocytes are known to mineralise calcium and therefore once the phenotype of these cells was determined the existence of mineralisation was less surprising.

However, it is of note that no one has ever made a human hypertrophioc chondrocyte cell-line before, despite its obvious advantages in understanding the mineralisation process symptomatic of the arthritis condition. Moreover, no one has thought to make such a cell-line for the study and/or production of joint tissue for use in skeletal repair and/or replacement studies and/or surgery. We therefore also present herein knowledge of, and methods relating to, the production of a human hypertrophic chondrocyte cell-line.

According to the invention there is provided a human hypertrophic chondrocyte cell-line.

In an embodiment of the invention said cell-line is of human foetal origin and also ideally comprises an oncogene and ideally a temperature sensitive oncogene so that at a first permissive temperature an active oncogene product is expressed and at a second non-permissive temperature expression of the active oncogene product is prevented. We have found that use of such an oncogene product provides for a cell-line which at the non-permissive temperature shows type X collagen expression, a marker for hypertrophic chondrocytes.

According to a further aspect of the invention there is provided a method for producing a human hypertrophic chondrocyte cell-line, which method comprises:
a) immortalising at least one human foetal chondrocyte cell using an immortalising agent; and
b) culturing said immortalising cell in order to produce a population of human foetal-derived chondrocyte cells.

Preferably the immortalising agent is an oncogene and more preferably still a temperature sensitive oncogene which at a non-permissive temperature results in enhanced expression of the phenotypic characteristics of the chondrocyte.

In a preferred embodiment of the invention immortalisation is achieved using conventional transfection techniques and preferably the immortalising agent is an immortalising gene such as an oncogene and more preferably still a temperature sensitive mutant of the immortalising oncogene SV40-T. More preferably still said transfection is undertaken using retroviral temperature sensitive oncogene transduction.

Ideally said method further involves culturing said immortalised cell at the non-permissive temperature of the oncogene.

According to an aspect of the invention there is provided hypertrophic chondrocytes, or at least one hypertrophic chondrocyte cell-line, for use in providing joint matrix tissue.

According to an aspect of the invention there is provided a method of producing joint matrix tissue for use in repair and/or replacement of damaged tissue comprising:
a) providing isolated hypertrophic chondrocyte cells, or at least one hypertrophic chondrocyte cell-line;
b) culturing said cells or cell-line under conditions that promote matrix growth; and
c) harvesting said cells or cell-line and/or said matrix tissue and/or selected parts thereof.

The invention will now be described by way of example only with reference to the following figures wherein.

Figure 1 shows an upregulation of Type II collagen expression in human articular chondrocytes.

Figure 2 shows the extracellular calcification achieved by these cells using a standard histological procedure (Von Kassa staining, 11) for the identification of calcification in tissue matrix.

Figure 3 depicts the immunocytochemically localised type X collagen (12) in these cells suggesting the expression in-vitro of characteristics expected of hypertrophic chondrocytes.

Figure 4 shows a femur derived from a human foetus which has been cultured for 14 days.

Figure 5 shows 3 chondrocyte cell-lines cloned from foetal femur when cultured either in the presence of 1,25(OH)2D3 (top row) or in the absence of 1,25(OH)2D3.

Figure 6 shows an actively mineralising monolayer culture of a chondrocyte cell-line derived from human foetal cells.

### Immortalisation of Articular Chondrocytes

Reference 13 is provided as a general method of preparing human articular chondrocytes for primary culture, but any published method will suffice. In short chondrocytes from sections of mature human tibia and femoral condyles from patient biopsies were enzymatically digested (14) and placed in tissue culture flasks (Costar Ltd, UK) in Eagle's minimum essential medium (Gibco Ltd, UK) containing antibiotics and L-glutamine as well as 10% heat inactivated foetal bovine serum.

Cultures were maintained in a humidified atmosphere of 5% CO₂ at 37°C and medium changed at regular intervals. The adherent cell population was then transfected with a temperature sensitive mutant of the simian virus-40 derived large tumour (T) antigen using retroviral transduction. Any standard method of transfection of this sequence (along with linkage to an appropriate promoter to drive its expression e.g. LTR promoter) would suffice, such as calcium phosphate DNA precipitation, electroporation or micro-injection, but retroviral transduction was chosen for its simplicity of use.

### Immortalisation of Human Foetal Tissue

Foetal rib or femur tissue was taken at 7-9 weeks of gestation and placed in tissue culture flasks (Costar Limited, UK) in Eagle's minimum essential medium (Gibco Co Limited, UK) containing antibiotics and L-glutamine as well as 10% heat inactivated foetal bovine serum.

Cultured cells were maintained in a humidified atmosphere at 5% CO₂ and 37°C and medium changed at regular intervals (2-3 days). As is usual for primary cultures of resected tissue, cells within the sample move out of from the body of the tissue, adhere to the flask surface, and replicate, potentially, to cover the whole surface of the tissue culture dish. After an initial period of, say, 2 weeks in primary culture. The cell population was transfected with a temperature sensitive mutant of the simium virus-40 derived large tumour (T) antigen using retroviral transduction.

### Culturing of the Immortalised Cells in Order to Produce a Homogenous Population of Cells

In short, amphotrophically packaged retroviral particles comprising this construct and a resistance marker to geneticin, G418 (kindly donated by Dr M O'Hare, Institute of Cancer Research, Royal Marsden Hospital, Lincoln's Inn, Sutton, Surrey, alternatively, essentially the same construct expressing a temperature sensitive mutant of the SV40-T oncogene was kindly donated by Professor Phil Gaillimore, CRC Laboratories, University of Birmingham, UK) was added to the medium together with polybrene (Sigma Chemicals) to a final concentration of 0.8mg/ml. The vital titre was adjusted to give a low transduction efficiency of 0.0002% producing an average of 20 immortalised cell colonies per flask, each colony derived from a single cell. Two hours after virus addition, the culture medium was replaced with fresh medium. Cultures were maintained at 33°C, the permissive temperature for the active form of the SV40-T oncogene product. Five days after transduction, geneticin was added to the medium (0.4mg/ml) for a further 10 days to eradicate cells which had not incorporated the retroviral vector.

### Differentiation of Said Cells

Between 14-20 days after transduction, individual colonies of replicating cells were identifiable. Clones were selected on the basis of being well separated from other replicating colonies, the cells in each colony numbering between 100-1000 cells.

These were picked by ring cloning and expanded up to near confluence in 75cm² flasks (Costar UK Ltd) which equated to approximately 22 divisions from a single cell, prior to freezing stocks down in aliquots. Samples were also used for cell characterisations and to determine that they possessed the ability to express articular chondrocyte specific markers. Expression was effected by exposing the cells to the oncogene's non-permissive temperature (39°C). Clones were derived from the routine method of single cell expansion.

### Experiments to Show Functional Characteristics of the Differentiated Articular Chondrocyte Cell-Line

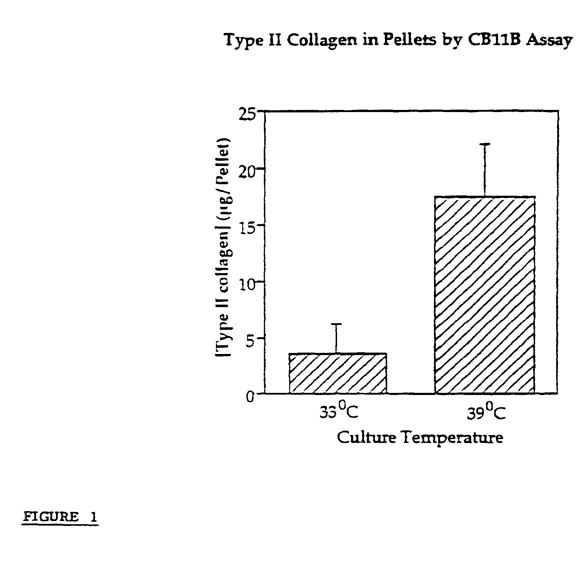

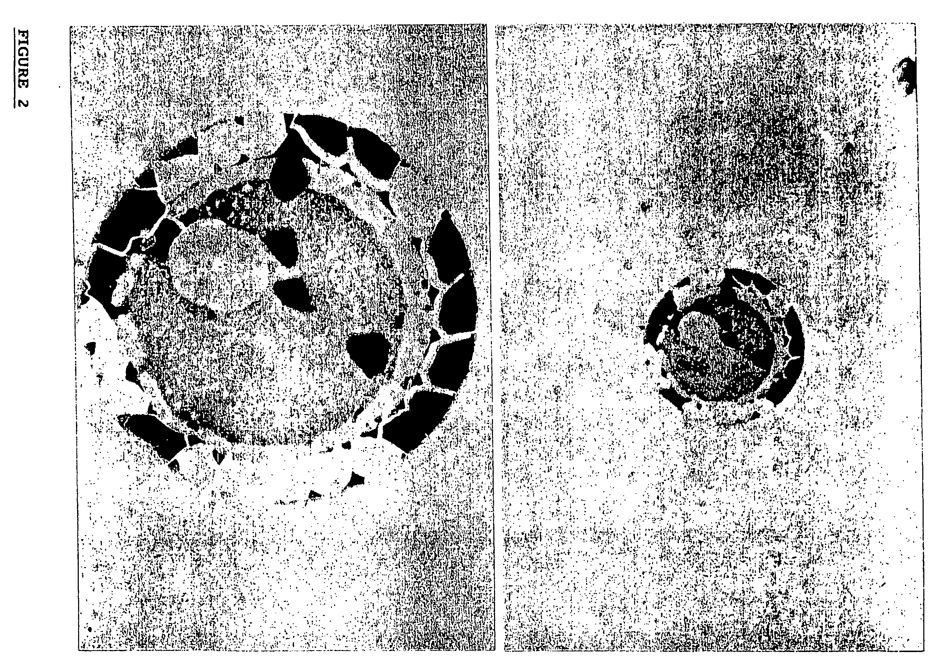

A specific marker of articular chondrocytes is the ability to express Type II collagen (8). The cells were therefore investigated in order to show Type II collagen expression. Immortalised human chondrocytes were exposed to 39°C, the non-permissive temperature of the oncogene, after pellet culture (9, 10) for six weeks. Figure 1 shows the mean of 6 pellets incubated at either the permissive temperature of 33°C or the non-permissive temperature of 39°C. The error bars represent the standard deviation. Figure 1 clearly shows an upregulation of Type II collagen expression at the non-permissive temperature of 39°C. This data indicates that our cell-lines comprise differentiated functional articular chondrocytes.

### Extracellular Calcification

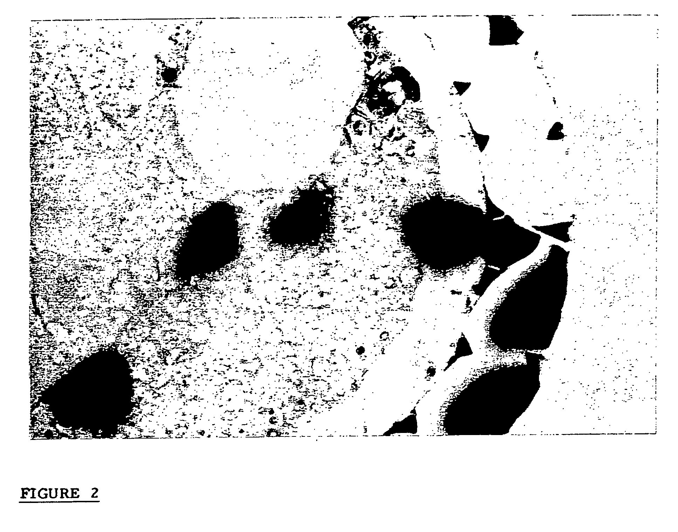

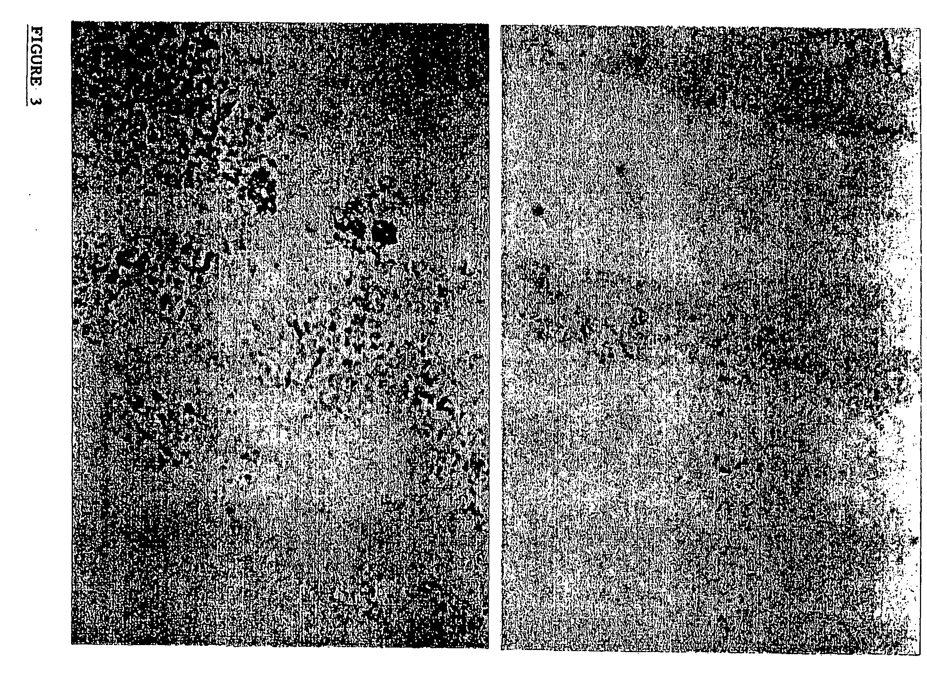

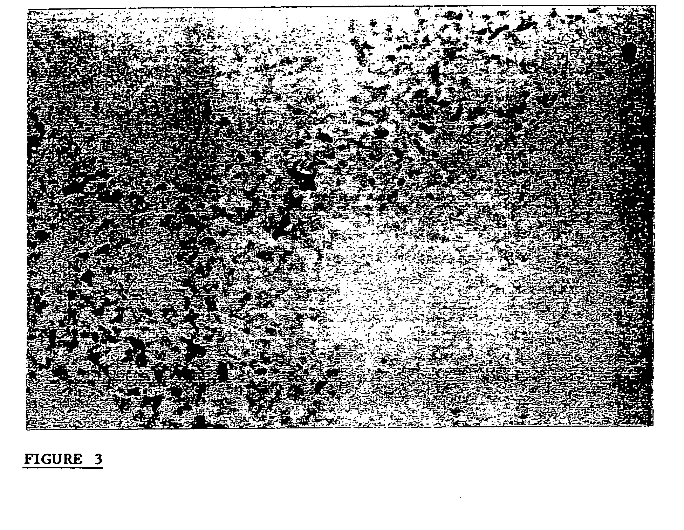

Figures 2 and 3 show the extracellular calcification achieved using articular chondrocyte cells.

Specifically, Figure 2 shows a standard histological procedure (von Kossa Staining, 11) for the identification of calcification in tissue matrix. The dark areas (Von Kossa Positive Staining) represent the calcified matrix produced by these articular chondrocytes, this being separated by the likely stained or unstained chondrocyte cells in the culture. Figure 3 is included to show immunocytochemically localised type X collagen (12) in these cells suggesting the expression in-vitro of characteristics expected of hypertrophic chondrocytes. This data tends to suggest that the cells of the invention may have de-differentiated in order to show characteristics associated with hypertrophic chondrocytes.

### Longevity of the Human Articular Chondrocyte Cell-Line

Our human articular chondrocyte cell-lines successfully differentiate to produce functional cells with articular chondrocyte characteristics. The cells continue to survive and have passed through more than 60 divisions over a period of 2 years producing 10¹⁸ cells from a variety of clones. The cell-lines continue to survive and moreover the cells of the line continue to show functional characteristics typical of the tissue type of the differentiated cell. These immortalised human articular chondrocyte cell-lines comprise differentiated cells and mineralise calcium.

### Experiments to Show Functional Characteristics of the Differentiated Hypertrophic Foetal-Derived Chondrocyte Cell-Line

A specific marker of hypertrophic chondrocytes is the ability to express type X collagen.

Our cells were therefore investigated in order to show type X collagen expression. All our foetal-derived chondrocytes expressed this cell marker and furthermore tests were undertaken and it was concluded that type I collagen was not expressed in these cells clearly showing that they were not of osteoprogenitor origin.

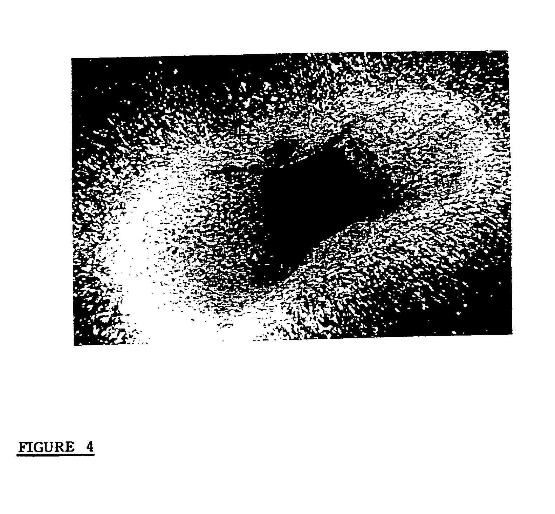

Figure 4 shows a femur derived from a human foetus of 7-9 weeks gestation and of approximately 600 micrometres in length. The femur has been cultured in the afore described culture medium for a period of 14 days to allow the outgrowth and expansion of replicating cell populations as would be the case for any primary cell culture. No prior treatment with enzyme(s) is required in this instance, however, to help in cell retrieval. Cell expansion for 14 days was performed prior to retroviral temperature sensitive oncogene transduction.

### Extracellular Calcification

The immortalised foetal-derived hypertrophic chondrocytes were also responsive to 1,25(OH)2D3 and expressed high levels of alkaline phosphatase activity, two further markers of the hypertrophic chondrocyte-like phenotype.

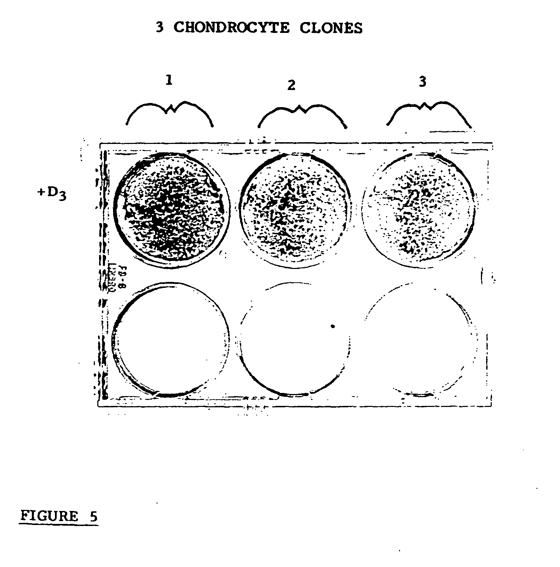

When the cells are left in monolayer culture for 10-14 days at 39°C, the oncogene's non-permissive temperature, and in the absence of added 3-glycerophosphate the cultures mineralise. This can be clearly seen in Figure 5 as it shows three chondrocyte cell-lines cloned from foetal femur after retroviral transduction with a temperature sensitive oncogene.

Each cell-line has a basal level of alkaline phosphatase activity and some type X collagen expression. If the cells are plated an incubated at the oncogene's non-permissive temperature, and in the presence of 1,25(OH)2D3, top row, each clone actively mineralises calcium as illustrated by the standard histological procedure of Von Kossa staining as afore described. Cells plated and incubated at the oncogene's non-permissive temperature in the absence of 1,25(OH)2D3, bottom row, do not actively mineralise calcium.

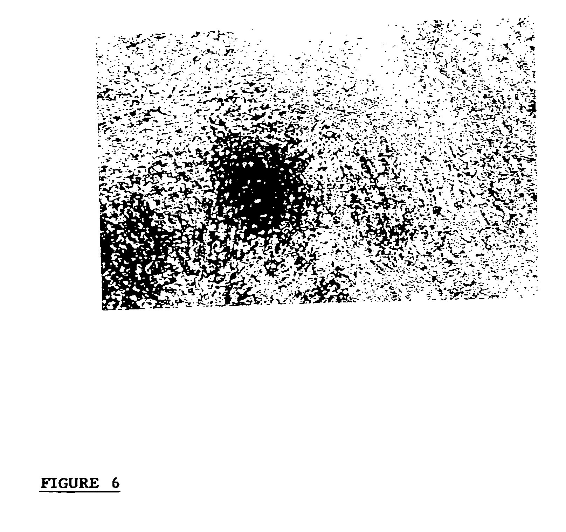

Finally, Figure 6 shows, again using Von Kossa staining, an actively mineralising monolayer culture of a chondrocyte cell-line derived from human foetal cells. Dark patches represent the mineralised matrix which develops and overlays the cultured cells which adhere the surface of the culture dish.

We have demonstrated that it is possible to provide human hypertrophic chondrocyte cell-lines for use in the development of biomaterials for use in any number of applications but particularly for use in developing materials for use in joint replacement.

### REFERENCES (Refs 1-7 deleted)

8. Hollander A. P., Heathfield T. F., Webber C, Iwata Y, Bourne R, Rorabeck C and Poole A. R., 1994. Increased damage to type II collagen in osteoarthritic articular cartilage detected by a new immunoassay. J Clin. Invest. 93:1722-1732.
9. Kata Y, Iwamoto M, Koike T, Suzuki F and Takano Y 1988. Terminal differentiation and calcification in rabbit chondrocyte cultures grown in centrifuge tubes: Regulation by transforming growth factor-b and serum factors. Proc. Nat. Acad. Sci (USA) 85:9552-9556.
10. Jikko A, Aoba T, Murakami H, Takano Y, Iwamoto M and Kato Y, 1993. Characterisation of the mineralisation process in cultures of rabbit growth plate chondrocytes. Dev. Biol. 156:372-380
11. Clark G, 1981. Miscellaneous Stains in Staining Procedures Eds. Williams and Wilkins. Publ Baltimore, New York.
12. Stephens M, Kwan A. P. L., Bayliss M, T., and Archer C. W., 1992. Human articular surface chondrocytes initiate alkaline phosphatase and type X collagen synthesis in suspension culture. J. Cell. Sci. 103:1111-1116.
13. Frazer A, Bunning R, Thavarajah M, Seid J and Russell R. G. G., 1994. Studies on type II collagen and aggrecan production in human articular chondrocytes in *vitro* and effects of transforming growth factor-b and interleukin-16. Osteoarthritis and Cartilage 2:235-245.
14. Gowen M, Wood D. D., Ihrie E. J., Meats E. J., and Russell R. G. G., 1984. Stimulation by human interleukin-1 of cartilage breakdown and production of collagenase and proteoglycanase by human chondroctes but not human osteoblasts in-vitro. Biochim Biophys. Acta 797:186-193.

## Claims

1. A human hypertrophic chondrocyte cell-line.

2. A cell-line according to Claim 1 wherein said cell-line is of foetal origin.

3. A cell-line according to Claims 1 or 2 wherein said cell-line comprises an oncogene.

4. A cell-line according to Claim 3 wherein said oncogene is temperature sensitive.

5. A cell-line according to Claims 3 or 4 wherein said oncogene is SV40T antigen.

6. A method for producing a human hypertrophic chondrocyte cell-line, which method comprises:
a) immortalising at least one human foetal chondrocyte cell using an immortalising agent; and
b) culturing said immortalised cell in order to produce a population of human foetal-derived chondrocyte cells.

7. A method according to Claim 6 wherein said immortalising agent is an oncogene.

8. A method according to Claim 7 wherein said oncogene is temperature sensitive.

9. A method according to Claim 7 or 8 wherein said oncogene is SV40T antigen.

10. A method according to Claims 8 or 9 wherein said method further involves culturing said immortalised cell at the non-permissive temperature of the oncogene.

11. An in vitro method of producing joint matrix tissue for use in repair and/or replacement of damaged tissue comprising:
a) providing isolated hypertrophic chondrocyte cells, or at least one hypertrophic chondrocyte cell-line;
b) culturing said cells or cell-line under conditions that promote matrix growth; and
c) harvesting said cells or cell-line and/or said matrix tissue and/or selected parts thereof.

12. The use of isolated hypertrophic chondrocytes, or at least one hypertrophic chondrocyte cell-line, in a method for producing joint matrix tissue.

13. A cell-line according to Claim 1 or 2 **characterised in that** it expresses type X collagen.

14. A cell-line according to Claim 13 **characterised in that** does not express type I collagen.

15. A cell-line according to Claim 4 or 5 **characterised in that** it expresses type X collagen at the non-permissive temperature of the oncogene.

## Patentansprüche

1. Humane hypertrophe Chondrozytenzelllinie.

2. Zelllinie nach Anspruch 1, die einen fötalen Ursprung hat.

3. Zelllinie nach Anspruch 1 oder 2, die ein Onkogen umfasst.

4. Zelllinie nach Anspruch 3, wobei das genannte Onkogen temperaturempfindlich ist.

5. Zelllinie nach Anspruch 3 oder 4, wobei das genannte Onkogen SV40T Antigen ist.

6. Verfahren zur Herstellung einer humanen hypertrophen Chondrozytenzelllinie, das die folgenden Schritte umfasst:
a) Immortalisieren von wenigstens einer humanen fötalen Chondrozytenzelle unter Verwendung eines Immortalisierungsmittels; und
b) Kultivieren der genannten immortalisierten Zelle, um eine Population humaner fötalstämmiger Chondrozytenzellen zu erzeugen.

7. Verfahren nach Anspruch 6, wobei das genannte Immortalisierungsmittel ein Onkogen ist.

8. Verfahren nach Anspruch 7, wobei das genannte Onkogen temperaturempfindlich ist.

9. Verfahren nach Anspruch 7 oder 8, wobei das genannte Onkogen SV40T Antigen ist.

10. Verfahren nach Anspruch 8 oder 9, das ferner das Kultivieren der genannten immortalisierten Zelle bei der nichtpermissiven Temperatur des Onkogens einschließt.

11. In-vitro-Verfahren zur Herstellung von Gelenkmatrixgewebe zur Verwendung bei Reparatur und/oder Austausch von beschädigtem Gewebe, umfassend die folgenden Schritte:
a) Bereitstellen von isolierten hypertrophen Chondrozytenzellen oder wenigstens einer hypertrophen Chondrozytenzelllinie;
b) Kultivieren der genannten Zellen oder Zelllinie unter Bedingungen, die ein Matrixwachstum fördern; und
c) Ernten der genannten Zellen oder Zelllinie und/oder des genannten Matrixgewebes und/oder ausgewählter Teile davon.

12. Verwendung von isolierten hypertrophen Chondrozyten oder wenigstens einer hypertrophen Chondrozytenzelllinie in einem Verfahren zur Herstellung von Gelenkmatrixgewebe.

13. Zelllinie nach Anspruch 1 oder 2, **dadurch gekennzeichnet, dass** sie Typ-X-Kollagen exprimiert.

14. Zelllinie nach Anspruch 13, **dadurch gekennzeichnet, dass** sie nicht Typ-I-Kollagen exprimiert.

15. Zelllinie nach Anspruch 4 oder 5, **dadurch gekennzeichnet, dass** sie Typ-X-Kollagen bei der nichtpermissiven Temperatur des Onkogens exprimiert.

## Revendications

1. Lignée cellulaire humaine de chondrocytes hypertrophiques.

2. Lignée cellulaire selon la revendication 1, dans laquelle ladite lignée cellulaire est d'origine foetale.

3. Lignée cellulaire selon les revendications 1 ou 2, dans laquelle ladite lignée cellulaire comprend un oncogène.

4. Lignée cellulaire selon la revendication 3, dans laquelle ledit oncogène est sensible à la température.

5. Lignée cellulaire selon les revendications 3 ou 4, dans laquelle ledit oncogène est un antigène T de SV40.

6. Méthode de production d'une lignée cellulaire humaine de chondrocytes hypertrophiques, laquelle méthode comprend :
a) immortaliser au moins une cellule chondrocyte foetale humaine en utilisant un agent d'immortalisation; et
b) cultiver ladite cellule immortalisée afin de produire une population de cellules chondrocytes dérivées de foetus humain.

7. Méthode selon la revendication 6, dans laquelle ledit agent d'immortalisation est un oncogène.

8. Méthode selon la revendication 7, dans laquelle ledit oncogène est sensible à la température.

9. Méthode selon la revendication 7 ou 8, dans laquelle ledit oncogène est un antigène T de SV40.

10. Méthode selon les revendications 8 ou 9, où ladite méthode comprend en plus cultiver ladite cellule immortalisée à la température non permissive de l'oncogène.

11. Méthode de production in vitro de tissu matriciel articulaire à utiliser dans la réparation et/ou le remplacement de tissu endommagé, comprenant :
a) fournir des cellules chondrocytes hypertrophiques isolées, ou au moins une lignée cellulaire de chondrocytes hypertrophiques;
b) cultiver lesdites cellules ou ladite lignée cellulaire dans des conditions qui favorisent la croissance de la matrice; et
c) récolter lesdites cellules ou ladite lignée cellulaire et/ou ledit tissu matriciel et/ou des parties sélectionnées de ceux-ci.

12. L'utilisation de chondrocytes hypertrophiques isolés, ou d'au moins une ligne cellulaire de chondrocytes hypertrophiques, dans une méthode de production de tissu matriciel articulaire.

13. Lignée cellulaire selon la revendication 1 ou 2, **caractérisée en ce qu'**elle exprime le collagène de type X.

14. Lignée cellulaire selon la revendication 13, **caractérisée en ce qu'**elle n'exprime pas le collagène de type I.

15. Lignée cellulaire selon la revendication 4 ou 5, **caractérisée en ce qu'**elle exprime le collagène de type X à la température non permissive de l'oncogène.
